# EUROPÄISCHE PATENTSCHRIFT

(11) **EP 2 045 320 B1**
(45) Veröffentlichungstag und Bekanntmachung des Hinweises auf die Patenterteilung: **25.04.2012**
(21) Anmeldenummer: 07116792.8
(22) Anmeldetag: 19.09.2007
(51) Int. Cl.: A61K 8/06, A61K 8/31, A61K 8/34, A61K 8/37, A61K 8/46, A61K 8/55, C11D 3/28, C11D 3/32, C11D 3/33, C11D 3/43, A61Q 1/00, A61Q 5/02, C11D 3/18, C11D 3/20, C11D 3/50, C11D 11/00, C11D 17/00, A61Q 3/04, C09D 9/00, C09D 9/04

(54) **Reinigungsmittel zur Entfernung von Farbschichten von Oberflächen, Verfahren zur Herstellung des Mittels und Verfahren zur Reinigung**
Cleaning agent for removing paint layers on surfaces, method for manufacturing the agent and cleaning method
Produit de nettoyage pour la suppression de couches de couleurs de surfaces, procédé de fabrication du produit et procédé de nettoyage

(43) Veröffentlichungstag der Anmeldung: 08.04.2009
(73) Patentinhaber: Bubbles & Beyond Gmbh, 04229 Leipzig (DE)
(72) Erfinder: Schumann, Dirk, 04229 Leipzig (DE); Surkow, Rainer, 04105 Leipzig (DE)
(74) Vertreter: Stolmár Scheele & Partner

(56) Entgegenhaltungen:
- EP-A- 0 525 892
- WO-A-91/00337
- WO-A-99/06520
- WO-A-2005/123028
- DE-A1- 10 049 831
- GB-A- 2 190 681
- US-A- 5 374 614
- US-A- 5 952 287
- US-A- 6 071 867
- US-A1- 2005 130 869
- US-A1- 2007 178 144

## Beschreibung

Die vorliegende Erfindung beschreibt neue Reinigungsmittel, deren Verfahren zur Herstellung und ein Verfahren zur Reinigung verschiedener Oberflächen, speziell wenn diese in erwünschter oder unerwünschter Weise mit Lacken und Farben, Farbsprays, Lack- bzw. Filzstiften oder anderen farblichen Oberflächenbeschichtungen behandelt wurden. Das erfindungsgemäße Reinigungsmittel dient insbesondere zur Entfernung von Graffiti von porösen Untergründen, zum Abbeizen von Lacken von Metall-, Holz- oder Glasoberflächen und zur Entfernung von Nagellack. Die Reinigungswirkung der erfindungsgemäßen Mittel besteht dabei in einer Fragmentierung der Farbschichten und deren Ablösen vom Untergrund, wobei ein Verschmieren weitgehend vermieden wird. Ermöglicht wird dies durch die erfindungsgemäße Zusammensetzung spezieller Mikroemulsionen.

Anfang der siebziger Jahre breitete sich, von New York ausgehend, im Zuge der Hip-Hop Kultur das Besprühen und Bemalen von Wänden und anderen Oberflächen aus. Nach einer Schätzung des Deutschen Städtetages (April 2002) beläuft sich der volkswirtschaftliche Schaden durch Graffiti in Deutschland auf etwa 200 Millionen Euro, hinzu kommen etwa 50 Millionen bei den U- und S-Bahnen und der Deutschen Bahn. Auch wenn die Meinungen darüber weit auseinander gehen und in vielen Fällen gut ausgearbeitete Graffiti (sog. "High-Graffiti" oder "Masterpiece") durchaus eine Kunstform darstellen, handelt es sich im deutlich überwiegenden Teil um Schmierereien (sog. "Tags") und damit um Sachbeschädigung. Die bisherigen kommerziell erhältlichen Graffitientferner helfen nur bedingt oder gar nicht. Sie sind meist nur auf glatten Flächen wirksam und benötigen eine lange Einwirkzeit von in der Regel 30 Minuten bis zu 2 Stunden. In gewissen Fällen wird sogar eine Einwirkzeit von bis zu 4 Stunden und eine anschließende Behandlung mit einem Hochdruckreiniger mit 90 °C heißem Wasser mit einem Druck von 150 bar empfohlen.

Die Anforderungen an einen Graffitientferner sind sehr hoch, da das Mittel auf unterschiedlichen Untergründen wirken muss und Graffitifarben zudem sehr uneinheitlich in ihrer Zusammensetzung sind. Die Graffiti-Farben und Beschichtungen besitzen keine einheitliche Zusammensetzung, d.h. die Bindemittel variieren je nach Herstellerfirma, dem Anwendungszweck der Farbe und selbst innerhalb von Artikelserien. Nähere Auskünfte der Herstellerfirmen zu den Bestandteilen ihrer Farbprodukte werden in der Regel nicht erteilt.

Die besprühten oder bemalten Untergründe sind meist aus Beton, Putz, Ziegel und Klinker, aber auch aus Feinkeramik und Natursteinen, wie Marmor oder Sandstein. Bei porösen Untergründen dringen die Farben in die Poren und Mauerfugen ein, so dass ein Entfernen nur schwer möglich ist. Einfacher zu reinigen sind glatte Oberflächen, wie lackierte oder unlackierte Metalle, Kunststoffe oder Glas.

Neben dem Untergrund entscheiden auch die Farbpigmente über den Aufwand der Farbentfernung. Zum einen hinterlassen dunkle Farbtöne bereits rein optisch einen auffälligen Farbschatten auf hellen Wänden, zum anderen werden feinere Pigmentpartikel leichter in Poren eingetragen und verbleiben dann darin. Farbschatten werden teilweise auch durch Pigmente mit ungenügender "Echtheit" erzeugt. Hier sind die Pigmente nicht völlig unlöslich, so dass Pigment-Moleküle z.B. in einen lackierten Untergrund oder durch einen Neuanstrich migrieren und führen u.a. zum sog. "Durchbluten".

Zu den feinteiligen Pigmenten gehören v.a. die Ruße (schwarz) und Phthalocyanine (grün bis blau) sowie einige andere organische Pigmente. Bei einfach aufgebauten organischen Pigmenten finden sich auch häufig solche, die zum Durchbluten neigen. In der Regel besitzen die weißen, gelben und roten Pigmente auf mineralischer Basis (Titandioxid, Eisenoxide etc.), der blaue Ultramarin sowie die Metallic-Pigmente (Aluminium, Bronzen) größere Partikeldurchmesser und lassen sich daher leichter aus dem Bindemittel herauslösen.

Die eingesetzten Bindemittel der Graffitifarben können Alkydharze sein mit unterschiedlichem Aufbau (z.B. Polyester- bzw. Fettsäureart und -gehalt), Harze, modifizierte oder vollsynthetische Kautschuke, Celluloseester, Aldehydkondensate oder viele anderen Polymerisate. Bei lösungsmittelfreien, wassermischbaren Sprayfarben sind es häufig Acryl- oder Polyvinylacetat-Dispersionen, manchmal auch alkalisch gelöste Acrylharze.

Die Graffitientfernung ist ein spezieller Fall der Lackentfernung und des Abbeizens. Die Aufgabe der Lackentfernung bei gleichzeitigem Schutz des Untergrundes ist in beiden Fällen gleich. Im Gegensatz zur Graffitientfernung ist die Lackentfernung oder das Abbeizen allerdings mit weniger schwierigen, d.h. porösen oder stark strukturierten Untergründen verbunden, dafür müssen hier oft auch vernetzte Lacksysteme oder koaleszierte Hochpolymerpartiken aus Dispersionsfarben entfernt werden, die sich durch eine Unlöslichkeit in Lösungsmitteln und in einer besonders guten Haftung zum Untergrund auszeichnen.

Seit Jahrzehnten wurden für die Lackentfernung universell wirkende Formulierungen verwendet, nämlich starke Lösungsmittel, allen voran Dichlormethan. Auf Grund der Toxizität dieses chlorierten Kohlenwasserstoffs und einem verstärkten Umweltbewusstsein der Gesellschaft sind diese Lösungsmittel und verwandte Kohlenwasserstoffe heutzutage nicht mehr ohne weiteres einsetzbar. So beschäftigen sich in Deutschland die Technischen Regeln für Gefahrstoffe TRGS 6123 eingehend mit möglichen Ersatzstoffen und Verfahren für Dichlormethan. Alternative halogenfreie Abbeiz-Formulierungen haben häufig den Nachteil, dass sie länger Einwirkungszeiten benötigen, teilweise hochgradig entflammbar sind und nicht bei jedem Lacksystem ein gleichermaßen gutes Ablösen zeigen. Gerade die letztgenannte Problemstellung zeigt sich heutzutage häufiger, da immer mehr wasserbasierte Lackformulierungen auf den Markt kommen, die gänzlich andere Solubilisierungseigenschaften erwarten, als die bisher verwendeten Lösungsmittelbasierten Lacksysteme. Für die heutige Vielzahl an Lacksystemen werden also Formulierungen zur Lackentfernung benötigt, die sowohl hydrophile wie auch hydrophobe Lösungseigenschaften aufweisen. Die Lackentfernung betrifft im übrigen nicht nur das Ablösen von aufgetrockneten Lackschichten, sondern auch die Entfernung von frischen Lackformulierungen, wie sie in weiten Anwendungsfällen auftreten, vom Malerhandwerk bis zur Druckfarbenindustrie.

Ein spezielles Gebiet der Lackentfernung ist die Entfernung von Nagellack. Bei der Entfernung von kosmetischen Nagellacken tritt infolge der Einwirkung von gebräuchlichen organischen Lösungsmitteln in den weitaus meisten Fällen eine Entfettung und ein Auslaugen der Nägel sowie der umliegenden Hautpartien auf. Dies macht sich in Form eines Weißwerdens von Nägeln und Haut bis hin zu brüchigen und glanzlosen Nägeln bei der Daueranwendung von Nagellackentferner bemerkbar. Seit vielen Jahrzehnten werden daher bereits verschiedene Vorschläge gemacht, um diese Effekte zu vermeiden oder gar zu verhindern. Obwohl es bekannt ist, dass die Entfettung und der Entzug von wichtigen Aufbaustoffen aus dem Nagel durch die Lösungsmittel erfolgt, wurden und werden immer noch Aceton, Ethylacetat, Butylacetat und ähnliche in Nagellackentfernern eingesetzt.

Um Graffiti zu entfernen, werden im Stand der Technik neben mechanischen Verfahren wie Sandstrahlen, Trockeneisstrahlen oder der Laserablation meist flüssige oder gelartige Graffitientferner verwendet. Der Vorteil der Graffitientferner ist die schnelle Möglichkeit eines Auftrags und die dann beginnende gleichzeitige Wirkung auf das Ablösen der Farbe auf größeren Flächen, während mit den mechanischen nur lokal die Farbe abgelöst werden kann. Die bisherigen Graffitientferner enthalten einzelne Lösungsmittel oder Lösungsmittelgemische, die die Aufgabe haben, die Bindemittel des Graffiti-Lacks physikalisch zu lösen. In einigen Graffitientfernern sind die Lösungsmittel in Wasser emulgiert. Die oft dünnflüssigen Lösungsmittel oder Emulsionen werden dabei gegen ein Ablaufen an senkrechten Oberflächen meist durch Additive verdickt.

Typisch für konventionelle Graffitientferner sind nachfolgende Lösungsmittel, die alle in der Liste der Technischen Regeln für Gefahrstoffe (TRGS 612, März 2002) zu finden sind. Häufig sind es Ester, wie z.B. kürzere und/oder längere Fettsäureester, wie z.B. Methyloleat bzw. Fettsäuregemische wie Rapsölfettsäuremethylester ("Biodiesel"), sowie verschiedene Dicarbonsäureester, z.B. sog. Dibasenester (DBE-Ester). EP 1772 496 A1 verwendet solche Methylester, wie auch Cyclohexanon, teilweise in (Mikro-)Emulsion zum Auflösen der Graffitifarben. Ab und zu werden auch Milchsäureester eingesetzt. Verbreitet ist auch die Verwendung von Glykolderivaten, wie Propylencarbonat, 1-Methoxy-2-propanol oder 2-Methoxy-1-methylethylacetat.

Strukturell ähnlich ist hier auch das in der Offenlegungsschrift DE 10 2004 015 092 A1 beschriebene Ethyl-3-ethoxypropionat. Ethyl-3-ethoxypropionat wurde aber auch bereits schon viele Jahre zuvor in DE 691 28 887 T2 (EP 0 551 378 B1) zur Aktivierung der stark polaren, aprotischen Lösungsmittel 1-Methyl-2-pyrrolidon (NMP) und Gamma-butyrolacton eingesetzt. Gerade das 1-Methyl-2-pyrrolidon ist ein bekanntes und ausgezeichnetes Lacklösemittel, welches entweder in reiner Form oder in Mischungen mit anderen Lösemitteln in vielen Abbeizer sowie Graffitientfernern eingesetzt wird. So beschreibt DE 695 21 333 T1 den Einsatz von 1-Methyl-2-pyrrolidon, aber auch anderen Pyrrolidonen in Mengen von 1 bis 60 Gew.-% in der Formulierung. Auch das dem Gamma-butyrolacton strukturell nahestehende Tetrahydrofuran wird in Graffitientfernern verwendet. Um 1-Methyl-2-pyrrolidon zu ersetzen wird in der Offenlegungsschrift DE 10 2004 012 751 A1 das 1-Ethyl-2-pyrrolidon beschrieben, welches auch bereits schon Bestandteil von DE 695 21 333 T1 war. Als strukturell sehr ähnlich zu den Pyrrolidonen kann auch das N-Methylcaprolactam aus Offenlegungsschrift DE 102004015182 A1 angesehen werden.

Dichlormethan und Gamma-butyrolacton sind aufgrund ihrer umwelt- bzw. gesundheitlich bedenklichen Wirkung zu vermeiden. Aber auch 1-Methyl-2-pyrrolidon ist in der jüngsten Zeit in Verdacht geraten teratogen zu wirken. Viele Graffitientferner und Abbeizer beinhalten oft größere Mengen an NMP, da es für die Entlackung hervorragende geeignet ist und bis vor kurzem als toxikologisch wenig bedenklich galt. Unabhängig davon, ob sich die Verdachtsmomente in Zukunft erhärten, ist sein vollständiger Ersatz auch aus Sicht der Produktvermarktung sinnvoll.

Kurzkettige Lösungsmittel-Moleküle dringen oft schnell in die Bindemittel der Graffiti-Farben ein, verdunsten in den meisten Fällen aber auch wieder schnell. Langkettige Lösungsmittel-Moleküle benötigen für diesen Vorgang häufig wesentlich mehr Zeit (z. B. zwischen etwa 20 Minuten bis 2 Stunden) und führen zu einem Anquellen der Beschichtungen, so dass diese danach mechanisch leichter vom Untergrund abgerieben werden können.

Nachteilig an einzelnen Lösungsmitteln und selbst bei Lösungsmittel-Gemischen ist, dass aufgrund der Komplexität des Lösevorgangs, welcher bis heute besonders im Hinblick auf das Lösen von Lacken oder Polymeren noch nicht komplett in seinen Einzelheiten verstanden ist, nicht alle Bindemittel-Typen gleichermaßen gut gelöst werden können. Dies macht sich dann bemerkbar, wenn man versucht verschiedene Graffiti-Farben mit demselben Entferner-System zu lösen. Aus einer breiten Palette von verschiedenen Aerosol-Farben lassen sich immer einige davon nicht zufriedenstellend vom Untergrund ablösen.

Selbst im Fall eines gelungenen Lösevorgangs besteht ein weiterer Nachteil von Lösungsmitteln darin, dass das Bindemittel der Graffiti-Farbe stark verdünnt und mitsamt den Farbpigmenten über den ursprünglichen Rand des Graffito hinaus verteilt wird. Sofern der Untergrund nicht glatt ist, sondern eine strukturierte Oberfläche besitzt, bleiben in vielen Fällen Reste des Bindemittels und v.a. die Farbpigmente in feinsten Ritzen und Poren hängen. Dies macht sich später in Form eines farbigen "Schattens" bemerkbar, der nur sehr schwer bis überhaupt nicht mehr zu entfernen ist. Der Untergrund muss wohlgemerkt dabei gar nicht porös und saugfähig wie die meisten Putze an Hauswänden sein, auch an glasierten rauen Kacheln, Keramiken oder Klinkern lässt sich diese Schattenbildung beobachten.

Da man bei zu entfernenden Graffiti-Farben am Schadensort nicht die Pigment-Bindemittel-Zusammensetzung erkennen kann, muss ein Graffitientferner ein möglichst breites Spektrum an Lösungsfähigkeit besitzen. Wie oben angemerkt können dies auch Lösungsmittel-Gemische nicht vollständig erzielen. Es muss also ein System gefunden werden, womit sich eine große Anzahl an unterschiedlichen Bindemitteln vom Untergrund ablösen lassen. Das System sollte die Graffitifarben in einer kurzen Zeit, z. B. innerhalb von 10 Minuten, maximal bis zu einer halben Stunde anlösen und vom Untergrund möglichst ohne Farbschatten entfernen können. Der Graffitientferner sollte keine giftigen oder langfristig gesundheits- bzw. umweltproblematischen Stoffe enthalten, namentlich keine organischen Halogen-Verbindungen (wie Dichlormethan), kein Gamma-Butyrolacton und möglichst auch keine N-Alkyl-pyrrolidone, aber auch keine starken Säuren oder Basen.

Ebenso wie bei Graffitientfernern sollten Abbeizer keine giftigen oder langfristig gesundheits- bzw. umweltproblematischen Stoffe enthalten, namentlich keine organischen Halogen-Verbindungen wie Dichlormethan, kein Gamma-Butyrolacton und möglichst auch keine N-Alkyl-pyrrolidone. Ebenso sollten starken Säuren oder Basen vermieden werden. Die breite Anwendbarkeit und die schnelle Wirkung gelten bei Dichlormethan-haltigen Abbeizern immer noch als vorteilhaft. Dichlormethanfreie Mittel benötigen jedoch oft deutlich längere Einwirkungszeiten, teilweise bis über 24 Stunden. Auch wenn dadurch andere größere Tiefenwirkung erzielt wird, sind solche langen Zeiträume inakzeptabel. Daneben bleibt das Problem des Verschmierens durch den Einsatz von Lösungsmitteln oder deren Gemischen. Ein geeigneter Abbeizer sollte daher nicht umwelt- und gesundheitsgefährdend sein, das heißt keines der oberen genannten Lösungsmittel, Säuren oder Basen enthalten, in möglichst kurzer Zeit auf eine sehr breite Palette an Farben und Bindemittel wirken, kein Verschmieren der Altlacke herbeiführen, die verschiedenen Untergründen nicht angreifen und möglichst mit Wasser entfernbar sein.

Wie im Fall der Graffiti-Entfernung und Lackentfernung ist auch bei der Nagellack-Entfernung - hier jedoch durch die kosmetische Anwendung im Besonderen - die Auswahl an möglichst wirkungsvollen einerseits und möglichst umwelt- und gesundheitsschonenden Inhaltsstoffen andererseits notwendig. Der Einsatz von Lösungsmitteln wie Dichlormethan (DE 1089515) oder Tetrachlormethan (DE 830094) bzw. ähnlich halogenierte Kohlenwasserstoffe ist in heutigen Formulierungen undenkbar. Aber auch über lange Zeit in Nagellackentfernern verwendete Lösungsmittel, wie z.B. gamma-Butyrolacton (GBL) existieren seit kurzer neue Erkenntnisse über ihre negativen Auswirkungen auf die Gesundheit. Der moderne Verbraucher erwartet von einem Nagellackentferner mittlerweile nicht nur die Funktionen der Reinigung und Pflege von Nägel und Hautpartien, sondern auch unbedenkliche und sensorisch dezente Inhaltsstoffe. Gerade in den letzten Jahren ist eine zunehmende Sensibilisierung der Verbraucher gegenüber den stechenden oder scharfen Gerüchen von Lösungsmitteln in einer Vielzahl von Produkten aufgetreten. Dies trifft insbesondere für die am häufigsten in Nagellackentfernern verwendeten Lösungsmittel zu, wie Aceton, Ethylacetat, Butylacetat, Ethyllactat, etc.
Auch wenn sich diese Lösungsmittel in Nagellackentfernern durch ihre schnelle Wirksamkeit und relativ geringe Toxizität bewährt haben, so ist doch ihr Ersatz auf Grund der charakteristischen Gerüche und der immer noch bestehenden Problematik der Haut- und Nagel-Entfettung dringend angeraten. Der mittlerweile in so genannten umweltverträglichen Produkten enthaltene Bio-Ethanol ist wiederum keine geeignete Alternative als Lösungsmittel, da sich nur spezielle Nagellacke damit entfernen lassen, so dass ein komplett neues Wirksystem gefunden werden muss, das alle oben genannte Ansprüche erfüllt.
Ein Nagellackentferner sollte also in der Lage sein, die aufgetragenen Beschichtungen schnell und sauber vom Nagel zu entfernen, ohne die oben genannten Schadensbilder zu verursachen. Stark entfettende Lösungsmittel sollten nicht oder nur in geringen Mengen eingesetzt werden, rückfettende und Feuchtigkeit spendende Substanzen sollten in ausreichender Menge zur Verfügung stehen, dürfen aber nicht die Entfernung des Lackes behindern oder verzögern. Wie im Fall von Aerosol-Farben und anderen Farbmitteln, mit denen Graffiti erzeugt werden, existieren eine Reihe von polymeren Lackbindemitteln die in den Formulierungen von Nagellack Anwendung finden. Ein geeigneter Nagellackentferner muss neben der Nagelpflege auch in der Lage sein, alle diese unterschiedlichen Lackzusammensetzungen gleichermaßen gut vom Untergrund abzulösen. Ebenso wie im Fall des Graffitientferners, jedoch in diesem Anwendungsfall von etwas geringerer Bedeutung, sollten beim Einsatz des Nagellackentferners die Farbmittel nicht verschmiert werden.

Aufgabe der vorliegenden Erfindung war es also, ein Reinigungsmittel für eine breite Palette baulicher Untergründe und auch für Fingernägel zur Verfügung zu stellen, das ein schnelles und gründliches Entfernen der Farbe oder des Lackes ohne Verschmieren gewährleistet. Das Reinigungsmittel sollte nicht umwelt- oder gesundheitsgefährdend sein und eine breite Palette an Farben und Lacken lösen.

Die Aufgabe der Erfindung wird durch ein Reinigungsmittel gemäß Anspruch 1 gelöst. Die Unteransprüche stellen bevorzugte Ausführungsformen dar.

Die erfindungsgemäßen Flüssigkeiten bestehen aus mindestens einer wasserunlöslichen Substanz (Lipophile), teilweise wasserlöslichen Substanzen (Amphiphile) und einer wässrigen Phase (Hydrophile). Mehrkomponentensysteme vom Typ Wasser, Öl, Tensid und ggf. Cotensid, die sich spontan bilden und als Mehrstoffsysteme in Erscheinung treten, sind als Mikroemulsionen bekannt. Eine Mikroemulsion ist eine klare bis opaleszente homogene Flüssigkeit aus zwei normalerweise nicht miteinander mischbaren Flüssigkeiten und einem Tensid. Häufig wird eine Kombination aus Tensid und Cotensid verwendet, wobei definitionsgemäß das Tensid mit den lipophileren Eigenschaften als das Cotensid bezeichnet wird [de.wikipedia.org/wiki/Mikroemulsion].
Durch die erfindungsgemäße weitere Zugabe von amphiphilen Substanzen gemäß Anspruch 1b, die nicht der hydrophil-hydrophoben Struktur oder Eigenschaften von Tensid bzw. Cotensid folgen, kann eine Erweiterung des einphasigen kolloiddispersen Bereichs der Mikroemulsion erreicht und eine Veränderung der Eigenschaften eingestellt werden. Diese Systeme werden im Sinne der Erfindung als fluide Nanophasensysteme (kurz: Nanophasen) bezeichnet.

Die Nanophasen der Erfindung enthalten neben den wasserunlöslichen Substanzen a), den teilweise wasserlöslichen (amphiphilen) Substanzen c), Tensiden und Wasser vor allem eine Acetoacetatverbindung CH₃COCH₂ COOR, in der R Methyl, Ethyl, Propyl oder i-oder n-Butyl bedeutet, vorzugsweise Ethyl. Es handelt sich bei dem bevorzugt eingesetzten Ethylacetoacetat , um amphiphile Substanzen, die sich nicht in Tensid oder Cotensid (Löslichkeit in Wasser 125 g/L; klassische Cotensid mit Kopf-Schwanz-Struktur: Hexanol 5,8 g/L) einteilen lassen, aber das einphasige kolloiddisperse Mischsystem deutlich erweitern.

Vorzugsweise ist die Acetoacetatverbindung von 2-25 Gew.-% und mehr bevorzugt von 10-24 Gew.-% im Reinigungsmittel enthalten, wobei sich diese Prozentangabe sowie alle Prozentangaben in der vorliegenden Anmeldung auf das Gesamtgewicht des Reinigungsmittels beziehen.

Erfindungsgemäß handelt es sich bei den wasserunlöslichen Substanzen a) um solche mit einer Wasserlöslichkeit kleiner 2 g pro Liter. Diese Substanzen müssen quellende und/oder lösende Eigenschaften im Hinblick auf Lackbindemittel oder Polymere aufweisen. Hierzu zählen z. B. Alkane (Benzine) und Cycloalkane (vorzugsweise Cyclohexan). Auch Aromaten wie Toluol, Xylole oder andere Alkylbenzole sowie Naphthaline kommen in Frage. Bevorzugt sind langkettige Alkansäureester, wie fette Öle und Fettsäuremethylester (Biodiesel). Auch Benzylacetat gehört erfindungsgemäß zu den eingesetzten wasserunlöslichen Substanzen. Aber auch Terpene, z. B. monocyclische Monoterpene mit Cyclohexangerüst, können Verwendung finden. Besonders bevorzugt sind hier Terpene aus Cirusfrüchten, wie Citronen- und/oder Orangenterpene bzw. das darin enthaltene Limonen. Die wasserunlöslichen Substanzen sind vorzugsweise von 1,5 - 30 Gew.-% im Reinigungsmittel enthalten.

Als weitere amphiphile Substanzen können erfindungsgemäß höhere Alkohole verwendet werden. Besonders bevorzugt sind hierbei vor allem Cotenside mit hydrophil-lipophilen Molekülanteilen wie z. B. die n- und i-Isomere von Butanol, Pentanol, Hexanol, Heptanol, Octanol, Nonanol, Decanol, Undecanol und Dodecanol. Weitere Amphiphile mit typischer Cotensid-Struktur sind z.B. Polyoxyethylenether der genannten Alkohole, wie z.B. Octanol(4)ethoxylat (Berol^{®} 840, Akzo Nobel), Nonyl-/Undecanol(4)ethoxylat (Berol^{®} 260), Ethylan^{®} 1005 (Akzo Nobel).

Bevorzugt sind auch Cycloalkanole, wie Cyclohexanol oder besonders bevorzugt Phenylalkohole wie Phenylmethanol (Benzylalkohol), 2-Phenylethanol und 3-Phenyl-1-propanol. Ester kurzkettiger Alkohole, wie z.B. Ethylacetat, Propylacetat oder die Butylacetate sind ebenso als Amphiphile einsetzbar, sofern sie nicht zu gering wasserlöslich sind. Geeignete Amphiphile sind auch die sog. Dibasenester, d.h. Ester von Dicarbonsäuren, v.a. deren Methylester, die noch ausreichend wasserlöslich sind. Diese Ester dienen häufig auch der Wirkungsverstärkung und senken gleichzeitig häufig die Kosten der Formulierung.

Ebenso können kurzkettige Fettsäuren, wie Hexan-, Heptan-, Octansäure und deren Alkali- oder Ammoniumsalze Verwendung finden. Besonders bevorzugt sind deren Salze von Ethanolaminen.

Die weiteren amphiphilen Substanzen sind vorzugsweise von 2 bis 40 Gew.-% in der erfindungsgemäßen Zusammensetzung enthalten.

Das Reinigungsmittel umfasst weiterhin anionische, kationische, amphotere und/oder nichtionische Tenside. In der folgenden Aufstellung werden einige bevorzugt geeignete Tenside genannt.

Als anionische Tenside können z. B. Alkali- oder Ammonium-Salze von langkettigen Fettsäuren, Alkyl(benzol)sulfonate, Paraffinsulfonate, Bis(2-ethylhexyl) sulfosuccinat, Alkylsulfate, wie v.a. Natriumdodecylsulfat und für spezielle Anwendungen, bei denen es z.B. auf einen Korrosionsschutz ankommt, teilweise auch Alkylphosphate (z.B. Phospholan^{®} PE 65, Akzo Nobel) eingesetzt werden. Als nichtionische Tenside können Polyalkylenoxid-modifizierte Fettalkohole, wie z.B. Berol^{®}-Typen (Akzo-Nobel) und Hoesch T-Typen (Julius Hoesch), sowie auch entsprechende Octylphenole (Triton-Typen) oder Nonylphenole (sofern letztere nicht in großen Mengen in die Umwelt freigesetzt werden). Ein besonderes Einsatzgebiet ermöglichen die Heptamethyltrisiloxane (z.B. Silwet^{®}-Typen, GE Silicones), als Mittel zur starken Erhöhung der Spreiteigenschaften der Flüssigkeiten bzw. zur deutlichen Absenkung der Grenzflächenspannung.

Als kationische Tenside können z.B. Kokosbis (2-hydroxyethyl-) methylammoniumchlorid oder Polyoxyethylen-modifiziertes Talkmethylammoniumchlorid Verwendung finden. Daneben ist auch der Einsatz diverser amphoterer Tenside möglich. Soll ein weiter ph-Bereich abgedeckt werden, so hat sich das Kokosdimethylaminoxid (Aormox® MCD, Akzo-Nobel) als geeignet herausgestellt.

Die Tenside sind im Reinigungsmittel vorzugsweise von 9 bis 16 Gew.-% enthalten, bezogen auf das Gesamtgewicht des Reinigungsmittels.

Die erfindungsgemäßen Reinigungsmittel können gegebenenfalls mit Hilfsstoffen versetzt werden, die die Anwendung erleichtern bzw. verbessern. Hierbei geht es z. B. um eine Verdickung zur Erleichterung des Auftrags des Reinigungsmittels auf den Untergrund, um eine Erniedrigung der Entzündlichkeit, eine geruchlichen Verbesserung, eine Wirkungsverstärkung, eine Kostenreduzierung der Wirkstoffe oder um einen Korrosionsschutz. Die Summe der Hilfsstoffe macht vorzugsweise nicht mehr als 20 Gew.-% der Formulierung aus, die Menge eines einzelnen Hilfsstoffes beträgt oft nicht mehr als 10 Gew.-%.
**a)** Verdickung: Bei den meisten Flächen eines Anwendungsfalls handelt es sich um senkrechte Wände, von denen niedrigviskose Flüssigkeiten vom Schadensort eines Graffito nach unten zu schnell ablaufen. Eine Verdickung der Flüssigkeiten ist also günstig, um das Ablaufen zu verzögern oder gar zu verhindern. Um die aus den Versuchen resultierenden niedrigviskosen Flüssigkeiten zu verdicken, können handelsübliche Verdickungsmittel eingesetzt werden. Besonders bevorzugt ist pyrogene Kieselsäure, wie z.B. Aerosil^{®} 380.
**b)** Flammschutz: Da der Graffitientferner einige leichtflüchtige Stoffe enthalten kann, ist ein Flammschutzmittel gegebenenfalls sinnvoll. Durch Zusatz von z.B. Triethylphosphat (TEP) oder Trioctylphosphaten, wie dem Tris(2-ethylhexyl)phosphat (TEHP), kann das Mittel selbsterlöschend eingestellt werden.
**c)** Geruchliche Verbesserung: Falls für den Endverbraucher die Formulierung geruchlich verbessert werden soll, so kann dies mit einer Reihe üblicher Geruchsstoffe vorgenommen werden. Als Beispiele seien Ester, wie Methylbutanoat (Ananas), Ethylmethanoat (Himbeere), Pentylethanoat (Banane), Pentylpentanoat (Apfel), Pentylbutanoat (Aprikose) Octylethanoat (Orange) genannt. Letzteres kann z. B. auch gut mit Orangenterpenen bzw. D-Limonen kombiniert werden, was ein Beispiel für einen lipophilen Wirkstoff in der erfindungsgemäßen Flüssigkeit darstellen kann. Auch andere Geruchsstoffe finden sich im Bereich der Terpene, wie z. B. Geraniol.

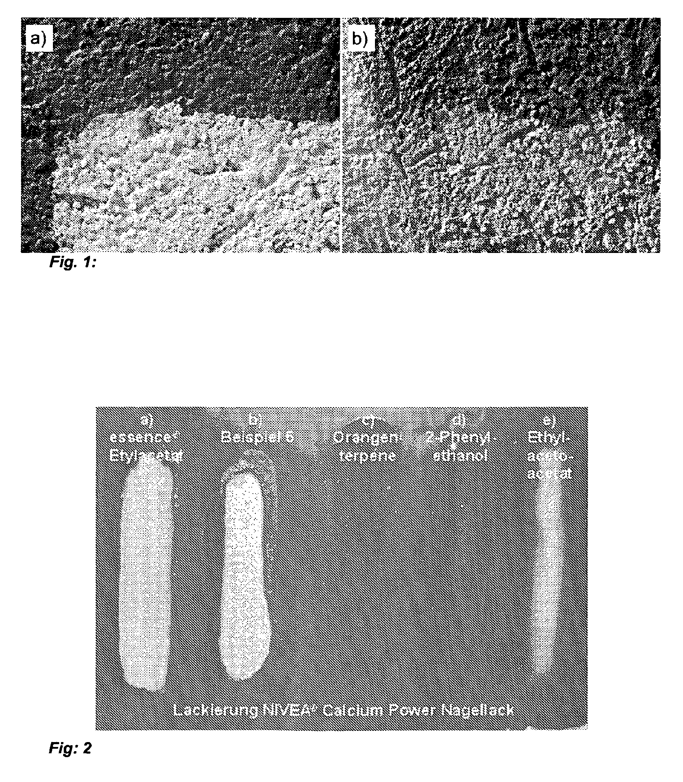

Die erfindungsgemäße Zusammensetzung, die eine optisch völlig transparente Flüssigkeit darstellt, löste überraschenderweise bestehend aus mindestens vier aber meist aus mehr Komponenten, löst überraschenderweise Lacke und Beschichtungen deutlich effektiver, sauberer und teilweise schneller von verschiedenen Untergründen ab, als
**a)** konventionelle Graffitientferner (vgl. Fig. 1), Abbeizer oder Nagellackentferner auf der üblichen Lösungsmittel-Basis bzw. als generell Lösungsmittel, selbst wenn diese als gut solvatisierend bekannt sind
**b)** die Einzelkomponenten für sich (vgl. Fig. 2)
**c)** Gemische der Lösungsmittel-Komponenten ohne Wasser und/oder ohne Tenside
**d)** konventionelle Graffitientferner auf bekannter Emulsions-Basis bzw. die aus den Lösungsmittel-Komponenten hergestellten Emulsionen
**e)** Gemische von Wasser und Tensiden.

Darüber hinaus lösten diese Flüssigkeiten die Graffiti-Farben mindestens ebenso gut vom Untergrund, wie die wirkungsvollsten Lösungsmittel der darüber hinaus getesteten Lösungsmittel, also namentlich z.B. Tetrahydrofuran, 1-Methyl-2-pyrrolidon, 1-Methoxy-2-propanol, Butyrolacton oder Dichlormethan.

Die erfindungsgemäßen Flüssigkeiten zeigten im Hinblick auf ihr Löseverhalten noch weitere überraschende Effekte:
**a)** der primäre Reinigungseffekt bestand nicht a priori in einem physikalischen Lösen des Lackes, da kein Lackbindemittel von den Flüssigkeiten molekulardispers gelöst wird, sondern in einem
**b)** starken Anquellen des Lacks, teilweise in wellenartiger Form, teilweise mit mikroskopisch kleinen Löchern in der Lackschicht und einem anschließendem Zerteilen (Fragmentieren) der Lackschicht in ca. 100 µm bis 2 mm große Lackstücke und
**c)** einem Unterwandern großer Bereiche der Lackschicht mit anschließendem Abheben der Schicht.
**d)** Alle behandelten, verschiedenartig strukturierten Untergründe zeigten praktisch keine Farbschatten, wenn der Lack mit feuchten Reinigungswerkzeugen oder Niederdruck-Spritzen (3 - 20 bar) behandelt wurde, da die Flüssigkeiten die Lacke nicht molekulardispers lösten, somit keine feinteiligen Pigmentpartikel freisetzten und in Verbindung mit Wasser als Waschlauge fungierten.

Nach einer Einwirkzeit von nur wenigen Sekunden bis Minuten konnten Lacke und Beschichtungen z.B. durch einen feuchten Schwamm, Bürste oder Pinsel vollständig abgelöst werden, wobei in Verbindung mit Wasser aus der vorher transparenten Flüssigkeit eine Emulsion entstand, die zwar ein deutlich geringeres Ablösevermögen, dafür aber eine ausgeprägte Waschwirkung besaß und die Farbpartikel bzw. Bindemittelreste vom Untergrund entfernte. Aufgrund dieser Waschwirkung entstand kein feiner farblicher Gradient ("Schatten"), den übliche Lösungsmittel(-Gemische) normalerweise vom Anwendungsort hin zu unbehandelten Stellen auf dem Untergrund hinterlassen und der aus verdünnten Bindemitteln und Pigmenten besteht.

Der Reinigungseffekt der erfindungsgemäßen Flüssigkeiten besteht also in zwei Stufen:
**a)** in unverdünnter Form: Anquellen oder Anlösen von Bindemittel-Systemen der Graffiti-Farbe meist in Verbindung mit einem Fragmentieren des Lackes - im Falle des Anquellens auch eine Unterwanderung der Farbschicht bis hin zum Untergrund
**b)** zusammen mit geringen Mengen Wasser, wie sie z.B. in einem feuchten Schwamm, Bürste oder Pinsel vorkommen: Bildung einer Waschlauge und Abheben der angequollenen und/oder aufgelösten Lackpartikel vom Untergrund.

Im Gegensatz zu bisherigen Lack-Entfernern auf üblicher Lösungsmittel-Basis ergibt sich auf den erfindungsgemäß behandelten Flächen also kein Farbgradient, d. h. ein Verschmieren der aufgelösten Graffitifarbe - weder zu den Rändern der Beschichtungen hin, noch in den Untergrund hinein. Die Fotographie in Fig. 1 von einem behandelten groben Rauputz-Untergrund zeigt diese Wirkung im Vergleich zu einem konventionellen Graffitientferner auf Basis von 1-Methoxy-2-propanol. Die Fotographie in Fig. 2 zeigt dieselbe Wirkung beim Entfernen von Nagellack

Im Gegensatz zu bisherigen Graffitientfernern auf üblicher Lösungsmittel-Basis zeigen die erfindungsgemäßen Systeme ein sehr breites Wirkungsspektrum auf das Ablösen der Farben bzw. Beschichtungen, ohne die Einschränkungen auf bestimmte Bindemittel-Typen, wie es häufig bei der Verwendung von Lösungsmitteln der Fall ist. Gegenüber Dispersionsfarben, die Farbüberzüge durch Koaleszens feinster, hochpolymerer Partikel erzeugen und häufig als Fassadenfarben eingesetzt werden, zeigt das erfindungsgemäße Graffitientferner-System eine Selektivität, d. h. hochwertige Fassadenfarben werden innerhalb der Einwirkungszeit nicht abgelöst. Im Fall des Abbeizers ist diese Selektivität nicht erforderlich, da hier auch Untergrund-Beschichtungen mit abgehoben werden müssen.

Im Gegensatz zu Lackentfernern auf üblicher Lösungsmittel-Basis zeigen die erfindungsgemäßen Systeme ein sehr schnelles Anquellen und Ablösen der Farben; je nach Alter, Untergrund und Farbe üblicherweise innerhalb von 10 Sekunden bis etwa 30 Minuten, vorzugsweise 20 Minuten, beim Graffitientferner, 20 Minuten bis 3 Stunden beim Abbeizer und 3 bis 30 Sekunden, vorzugsweise 3 bis 20 Sekunden, beim Nagellackentferner.

Gegenstand der Erfindung ist deshalb auch das Verfahren zur Entfernung unerwünschter Farben und Lacke von Oberflächen nach Anspruch 11.

Der Lackentferner wird hergestellt, indem in einem geeigneten Gefäß zunächst Wasser bzw. das Lösungsmittel mit Hydroxy-Funktionalität vorgelegt und dann das Tensid unter Rühren aufgelöst wird. Hierbei ist zu beachten, dass einige Tenside bereits Wasser in Lieferform enthalten können, so dass die in der Rezeptur vorausberechnete Wassermenge ggf. korrigiert werden muss. Beim Auflösen des Tensids muss darauf geachtet werden, dass der Lufteintrag in die Lösung so gering wie möglich gehalten wird, um ein übermäßiges Schäumen zu vermeiden. Für die großtechnische Realisierung gibt es bereits viele Variationen an Rührwerken und Rührer, um ein Schäumen weitgehend zu vermeiden. Die Rührgeschwindigkeit sollte bei Verwendung von Propellerrührern und idealen Verhältnissen von Rührerdurchmesser und Gebindedurchmesser üblicherweise 200 Umdrehungen pro Minute nicht überschreiten. Weiterhin muss darauf geachtet werden, dass einige (konzentrierte) Tenside bei Zugabe von Wasser Gele bilden können, die ein Rühren und eine weitere Verteilung erschweren können. In solchen Fällen müssen ggf. die wasserunlöslichen Substanzen (Ölphase) zuerst oder parallel zum Tensid zugegeben werden. Ein Schäumen kann auch durch die nachfolgende Zugabe der Ölphase verhindert werden, da diese oft eine gewisse Entschäumerwirkung besitzen. Nach Zugabe der Ölphase ist eine milchig-trübe Emulsion entstanden, die durch die Zugabe der weiteren amphiphilen Substanz mit Tensidstruktur (z.B. Alkanol), spätestens aber nach Zugabe des Amphiphils nach Anspruch 1b (z.B. der Acetoacetat-Verbindung) aufklart und schließlich in eine optisch transparente Mikroemulsion oder ein Nanophasensystem übergeht. Am Ende können noch Zusatzstoffe, wie z.B. flammhemmende Mittel (z.B. Triethylphosphat), Verdickungsmittel (z.B. Aerosile) und/oder andere Hilfsmittel zugefügt werden.

Gegenstand der Erfindung ist deshalb auch das Herstellungsverfahren gemäß Anspruch 10. Des weiteren betrifft die Erfindung die Verwendung der beschriebenen Reinigungsmittel als Graffitientferner, Abbeizer oder Nagellackentferner.

Es zeigen

### Fig. 1:

Vergleich des erfindungsgemäßen Graffitientferners nach Beispiel 2 mit einem konventionellen Graffitientferner auf Basis von 1-Methoxy-2-propanol (Baufan RICO Graffiti-Killer)

Fotografische Aufnahme einer mit Graffiti beschädigten Wand mit grobem Rauputz (Alter des Graffito mindestens 1 Jahr). Gleichzeitige und gleichlange Einwirkung der Graffitientferner, nach 5 Minuten Abwischen der Entferner mit einem Schwamm und anschließendes Abspülen der Wand mit einem Niederdruck-Wasserstrahl (< 3 bar):
a) Die erfindungsgemäße Formulierung zeigt keine Farbschatten, keine Beeinträchtigung der ursprünglichen Fassadenfarbe (weiß) und keinen Farbgradienten an den Rändern, sondern eine scharfe Abgrenzung zur unbehandelten Fläche.
b) Der konventionelle Graffitientferner auf Basis von 1-Methoxy-2-propanol zeigt dagegen eine schlechtere Farbentfernung mit unscharfen Rändern vom Wirkungsort zur unbehandelten Fläche, was durch ein Verschmieren der Graffiti-Farbe verursacht wird. Zudem ist die durch das Mittel verdünnte Farbe teilweise von den Poren des Putzes aufgenommen worden, was Farbschatten erzeugt, die nachträglich nur schwer zu entfernen sind.

### Fig. 2:

Wirksamkeit des konventionellen Nagellackentferners essence® Ethylacetat, des erfindungsgemäßen Nagellackentferners des Beispiels 5 und von Einzelkomponenten
a) gute Lackentfernung, Verschmierung zum Rand hin
b) gute Lackentfernung, scharfe Ränder mit gequollenen Bereichen
c) nahezu kein Ablösen, kein Anquellen
d) kaum Lackentfernung
e) Lackentfernung mit starkem Verschmieren zum Rand hin

Folgende Ausführungsbeispiele sollen die Erfindung näher erläutern, ohne sie darauf einzuschränken.

### Ausführungsbeispiele

### Beispiel 1: Graffitientferner

| **Stoff** | **Masseprozent [%]** |
|---|---|
| Wasser demineralisiert | 20,00 |
| Triethylphosphat | 4,50 |
| Ethylacetoacetat | 17,20 |
| Orangenterpene | 30,00 |
| Natriumlaurylsulfat | 12,30 |
| 1-Hexanol | 16,00 |
| | 100,00 |

Der Graffitientferner aus Beispiel 1 wurde hergestellt, indem in einem geeigneten Gefäß zunächst Wasser vorgelegt und darin das Tensid (Natriumlaurylsulfat) unter Rühren aufgelöst wurde. Beim Auflösen des Tensids sollte der Lufteintrag in die Lösung so gering wie möglich gehalten werden. Ein Schäumen kann durch die nachfolgende Zugabe der Orangenterpene verhindert werden, da diese eine gewisse Entschäumerwirkung besitzen. Nach diesem Schritt ist eine milchig-trübe Emulsion entstanden, die durch die Zugabe von 1-Hexanol und Ethylacetoacetat klar wird und schließlich in ein völlig transparentes Nanophasensystem übergeht. Am Ende wird das Triethylphosphat zugefügt.

### Beispiel 2: Graffitientferner

| **Stoff** | **Masseprozent [%]** |
|---|---|
| Wasser demineralisiert | 24,50 |
| Triethylphosphat | 5,00 |
| Ethylacetoacetat | 14,50 |
| Orangenterpene | 27,50 |
| Natriumolefinsulfonat (Hansanyl OS) | 12,50 |
| 1-Hexanol | 16,00 |
| | 100,00 |

Der Graffitientferner aus Beispiel 2 wurde verfahrenstechnisch analog Beispiel 1 hergestellt.

### Beispiel 3: Graffitientferner

| **Stoff** | **Masseprozent [%]** |
|---|---|
| Wasser demineralisiert | 32,00 |
| Triethylphosphat | 4,50 |
| Ethylacetoacetat | 11,50 |
| n-Butylacetat | 8,00 |
| Orangenterpene | 12,00 |
| Benzylacetat | 8,00 |
| Natriumdodecylsulfat | 11,00 |
| 1-Hexanol | 13,00 |
| | 100,00 |

Der Graffitientferner aus Beispiel 3 wurde verfahrenstechnisch analog Beispiel 1 hergestellt.

### Beispiel 4: Abbeizer

| **Stoff** | **Masseprozent [%]** |
|---|---|
| Wasser demineralisiert | 30,77 |
| Triethylphosphat | 4,32 |
| Ethylacetoacetat | 11,06 |
| Dibasen Ester (DuPont) | 7,69 |
| Benzylacetat | 7,69 |
| Orangenterpene | 15,39 |
| Hoesch NAS (als 100%-ige Substanz) | 10,58 |
| 1-Hexanol | 12,50 |
| | 100,00 |

Der Abbeizer aus Beispiel 4 wurde verfahrenstechnisch analog Beispiel 1 hergestellt.

### Beispiel 5: Nagellackentferner

| **Stoff** | **Masseprozent [%]** |
|---|---|
| Wasser demineralisiert | 43,49 |
| Ethylacetoacetat | 22,26 |
| 2-Phenylethanol | 14,84 |
| Ätherisches Öl Orangen (Duftstoff) | 2,45 |
| Natriumlaurylsulfat | 16,96 |
| | 100,00 |

Der Nagellackentferner aus Beispiel 5 wurde hergestellt, indem das Tensid (Natriumlaurylsulfat) unter leichtem Rühren bei Rührgeschwindigkeiten von 100 Umdrehungen pro Minute in eine Mischung aus Wasser, Ethylacetoacetat und 2-Phenylethanol eingerieselt wurde. Nach Bildung des Nanophasensystems wurde der Duftstoff hinzugefügt.

Die folgende Übersicht zeigt die Wirkung der erfindungsgemäßen Graffitientferner im Vergleich zu konventionellen Graffitientfernern bzw. Lösungsmitteln

**Tabelle 1: Wirkung der in den Beispielen 1 und 3 hergestellten Graffitientferner der Erfindung innerhalb von 5 Minuten Einwirkzeit**

| | | **Beispiel** | **Beispiel** | **Graffitientferner auf Basis** | **2-Butanol** |
|---|---|---|---|---|---|
| **Farbe** | **Untergrund** | **1** | **3** | **1-Methoxy-2-propanol** | |
| schwarz | Feinstein rau, mikroporös | vollständig | vollständig | Farbschatten | Kaum Entfernung |
| schwarz | | vollständig | vollständig | keine Entfernung in Poren | Keine Entrfernung in Poren |
| schwarz | | vollständig | Vollständig | vollständig | Kaum Entfernung |
| schwarz | | vollständig | Vollständig | Farbgradient | Zu ca. 80% Entfernung |
| schwarz | | vollständig | Vollständig | vollständig | Kaum Entfernung, Schmieren |
| rot | | vollständig | Vollständig | Farbschatten | Kaum Entfernung |
| dunkelrot | Rauhputz weiss gestrichen | vollständig | Vollständig | Farbschatten | Kein Ergebnis vorhanden |
| gelb | | vollständig | Vollständig | fast vollständig | |
| dunkelgrün | | vollständig | Vollständig | | |
| | | vollständig | Vollständig | | |
| türkisgrün | | vollständig | Vollständig | | |
| dunkelblau | | vollständig | Vollständig | | |
| blau | | vollständig | Vollständig | | |
| blau | | vollständig | Vollständig | | |
| gold metallic | | vollständig | Vollständig | | Fast vollständig |
| silber metallic | | vollständig | Vollständig | | Fast vollständig |

**Tabelle 2: Wirkung des erfindungsgemäßen Nagellackentferners nach Beispiel 5 nach einmaligem Auftrag des Entferners mit Watte-Pads und anschließendem Abwaschen der Nägel mit Wasser**

| **Nagellack** | | **Beispiel** | | **"essence pocket beauty"** |
|---|---|---|---|---|
| **Farbe** | **Marke** | **5** | **Aceton** | **Ethylacetat** |
| dunkelviolett | BIG BLUE | nach 3 Sekunden Ablösen | vollständig, jedoch mit Farbschmieren an Haut | nach 3 Sekunden auflösen, Farbschmieren an Haut |
| braun-gold | BIG BLUE | nach 3 Sekunden Ablösen, vollständig nach ca. 10 Sekunden | keine vollständige Entfernung des Lacks von Haut und Nagel | nach 3 Sekunden auflösen, Farbschmieren an Haut |
| rosa | essence cosnova | Nach ca. 10 Sekunden vollständig | vollständig | vollständig |
| orange | 60 seconds, Rimmel | nach 15 Sekunden Anlösezeit, dann schnell und vollständig | vollständig | vollständig |
| dunkelrot | Jade-Maybelline | sofort löslich, ohne Rückstände entfernbar | verschmieren | vollständig, aber mit verschmieren |
| dunkelrot | NIVEA | vollständig | verschmieren | vollständig |
| weiß | Luminelle, Yves Rocher | nach 10 Sekunden Anlösezeit, dann innerhalb 5 Sekunden schnell und vollständig | fast vollständig | vollständig |

## Patentansprüche

1. Reinigungsmittel zur Entfernung unerwünschter Farben und Lacke von Oberflächen, **dadurch gekennzeichnet, dass** das Reinigungsmittel eine Mikroemulsion oder ein fluides Nanophasensystem darstellt und die folgenden Bestandteile umfasst:
a) mindestens eine wasserunlösliche Substanz
b) eine amphiphile Substanz, die eine Acetoacetatverbindung CH₃COCH₂COOR ist, in der R C₁-C₄-Alkyl bedeutet
c) eine weitere amphiphile Substanz
d) mindestens ein anionisches, kanonisches, amphoteres und/oder nicht-ionisches Tensid
e) Wasser und/oder ein wasserlösliches Lösungsmittel mit Hydroxy-Funktionalität
und gegebenenfalls Hilfsstoffe.

2. Reinigungsmittel nach Anspruch 1, **dadurch gekennzeichnet, dass** die wasserunlösliche Substanz eine Wasserlöslichkeit von < 2 g pro Liter aufweist und die Substanz aus der Gruppe umfassend Alkane, Cykloalkane, Aromaten, langkettige Alkansäureester, Ester von Di- oder Tricarbonsäuren, Terpene, oder deren Mischungen ausgewählt ist.

3. Reinigungsmittel nach Anspruch 1 oder 2, **dadurch gekennzeichnet, dass** die wasserunlösliche Substanz ein monocyclisches Monoterpen mit Cyclohexangerost ist, vorzugsweise Orangenterpen, Cibronenterpen oder Limonen.

4. Reinigungsmittel nach einem der Ansprüche 1 bis 3, **dadurch gekennzeichnet, dass** es 1,5 bis 30 Gew.-% der wasserunlöslichen Substanz aufweist, wobei sich die Prozentangaben auf das Gesamtgewicht des Reinigungsmittels beziehen.

5. Reinigungsmittel nach einem der Ansprüche 1 bis 4, **dadurch gekennzeichnet, dass** die Acetoacetatverbindung von 2 bis 25 Gew.-% im Reinigungsmittel enthalten ist, wobei sich die Prozentangaben auf das Gesamtgewicht des Reinigungsmittels beziehen.

6. Reinigungsmittel nach einem der Ansprüche 1 bis 5, **dadurch gekennzeichnet, dass** die Acetoacetatverbindung Ethylacetoacetat ist.

7. Reinigungsmittel nach einem der Ansprüche 1 bis 6, **dadurch gekennzeichnet, dass** die weitere amphiphile Substanz eine Wasserlöslichkeit von 2 bis 128 g pro Liter aufweist und die Substanz aus der Gruppe umfassend C₄-C₁₂-Alkohole, Cycloalkanole, Phenylalkohole, kurzkettige Fettsäuren oder deren Alkali- oder Ammoniumsalze ausgewählt ist.

8. Reinigungsmittel nach einem der Ansprüche 1 bis 7, **dadurch gekennzeichnet, dass** die weitere amphiphile Substanz von 2 bis 40 Gew.-% im Reinigungsmittel enthalten ist, wobei sich die Prozentangaben auf das Gesamtgewicht des Reinigungsmittels beziehen.

9. Reinigungsmittel nach einem der Ansprüche 1 bis 8, **dadurch gekennzeichnet, dass** das Tensid nach Anspruch 1d) von 9 bis 16 Gew.-% im Reinigungsmittel enthalten ist, wobei sich die Prozentangaben auf das Gesamtgewicht des Reinigungsmittels beziehen.

10. Verfahren zur Herstellung eines Reinigungsmittels zur Entfernung unerwünschter Farben und Lacke von Oberflächen, **dadurch gekennzeichnet, dass** Wasser oder ein Lösungsmittel mit Hydroxy-Funktionalität vorgelegt und ein anionisches, kationisches, amphoteres und/oder nicht ionisches Tensid bei 10 bis 90 °C unter Rühren darin aufgelöst wird, wasserunlösliche Substanz(en) parallel oder nach Tensid-Zugabe zugesetzt werden und dann die entstandene Emulsion durch die Zugabe einer weiteren amphiphilen Substanz und einer Acetoacetatverbindung in eine optisch transparente Mikroemulsion oder ein Nanophasensystem überführt wird und am Ende des Mischungsvorgangs gegebenenfalls Hilfsstoffe zugefügt werden.

11. Verfahren zur Entfernung unerwünschter Farben und Lacke von Oberflächen, **dadurch gekennzeichnet, dass** das Reinigungsmittel nach einem der Ansprüche 1 bis 9 auf die unerwünschte Farbe oder den Lack aufgebracht wird, einwirkt und anschliessend die Farbe oder der Lack mit Wasser entfernt wird, wobei die Einwirkzeit beim Graffitientferner von etwa 10 Sekunden bis etwa 30 Minuten beträgt, beim Abbeizer von etwa 20 Minuten bis etwa 3 Stunden und beim Nagellackentfemen von etwa 3 bis etwa 30 Sekunden.

12. Verwendung des Reinigungsmittels nach den Ansprüchen 1 bis 9 als Graffitientferner, Abbeizer oder Nagellackentferner.

## Claims

1. Cleaning agent for removing unwanted paints and lacquers from surfaces, **characterized in that** the cleaning agent represents a microemulsion or a fluid nanophase system and includes the following constituents:
a) at least one water-insoluble substance
b) an amphiphilic substance which is an acetoacetate compound CH₃COCH₂COOR in which R represents C₁-C₄ alkyl
c) a further amphiphilic substance
d) at least one anionic, cationic, amphoteric and/or non-ionic surfactant
e) water and/or a water-soluble solvent with hydroxy functionality
and optionally excipients.

2. Cleaning agent according to claim 1, **characterized in that** the water-insoluble substance has a water solubility of < 2 g per litre and the substance is selected from the group comprising alkanes, cycloalkanes, aromatics, long-chained alkanoic acid esters, esters of di- or tricarboxylic acids, terpenes or mixtures thereof.

3. Cleaning agent according to claim 1 or 2, **characterized in that** the water-insoluble substance is a monocyclic monoterpene with a cyclohexane skeleton, preferably orange terpene, lemon terpene or limonene.

4. Cleaning agent according to one of claims 1 to 3, **characterized in that** it contains 1.5 to 30 wt.-% of the water-insoluble substance, wherein the percentages relate to the overall weight of the cleaning agent.

5. Cleaning agent according to one of claims 1 to 4, **characterized in that** the cleaning agent contains 2 to 25 wt.-% acetoacetate compound, wherein the percentages relate to the overall weight of the cleaning agent.

6. Cleaning agent according to one of claims I to 5, **characterized in that** the acetoacetate compound is ethyl acetoacetate.

7. Cleaning agent according to one of claims 1 to 6, **characterized in that** the further amphiphilic substance has a water solubility of 2 to 128 g per litre and the substance is selected from the group comprising C₄-C₁₂ alcohols, cycloalkanols, phenyl alcohols, short-chained fatty acids or their alkali or ammonium salts.

8. Cleaning agent according to one of claims 1 to 7, **characterized in that** the cleaning agent contains 2 to 40 wt.-% further amphiphilic substance, wherein the percentages relate to the overall weight of the cleaning agent.

9. Cleaning agent according to one of claims 1 to 8, **characterized in that** the cleaning agent contains 9 to 16 wt.-% surfactant according to claim 1d), wherein the percentages relate to the overall weight of the cleaning agent.

10. Method of producing a cleaning agent for removing unwanted paints and lacquers from surfaces, **characterized in that** water or a solvent with hydroxy functionality is introduced first and an anionic, cationic, amphoteric and/or non-ionic surfactant is dissolved therein at 10 to 90 °C accompanied by stirring, water-insoluble substance(s) are added parallel to or after the addition of surfactant and then the resulting emulsion is then transformed into an optically transparent microemulsion or a nanophase system by the addition of a further amphiphilic substance and an acetoacetate compound and at the end of the mixing process excipients are optionally added.

11. Method for removing unwanted paints and lacquers from surfaces, **characterized in that** the cleaning agent according to one of claims 1 to 9 is applied to the unwanted paint or the lacquer, takes effect in and then the paint or the lacquer is removed with water, wherein the exposure time is approximately 10 seconds to approximately 30 minutes in the case of the graffiti remover, approximately 20 minutes to approximately 3 hours in the case of the paint stripper and approximately 3 to approximately 30 seconds in the case of the nail-varnish remover.

12. Use of the cleaning agent according to claims 1 to 9 as graffiti remover, paint stripper or nail-varnish remover.

## Revendications

1. Détergent pour l'élimination de peintures et de laques indésirables de surfaces, **caractérisé en ce que** le détergent présente une microémulsion ou un système de nanophases fluide et comprend les constituants suivants :
a) au moins une substance insoluble dans l'eau
b) une substance amphiphile qui est un composé d'acétoacétate CH₃COCH₂COOR, dans lequel R signifie alkyle en C₁-C₄
c) une autre substance amphiphile
d) au moins un tensioactif anionique, cationique, amphotère et/ou non ionique
e) de l'eau et/ou un solvant hydrosoluble à fonction hydroxy
et éventuellement des adjuvants.

2. Détergent selon la revendication 1, **caractérisé en ce que** la substance insoluble dans l'eau présente une solubilité dans l'eau < 2 g par litre et la substance est sélectionnée parmi le groupe comprenant des alcanes, cycloalcanes, aromates, esters d'acide alcanoïque à longue chaîne, esters d'acides di- ou tricarboxyliques, terpènes ou leurs mélanges.

3. Détergent selon la revendication 1 ou 2, **caractérisé en ce que** la substance insoluble dans l'eau est un monoterpène monocyclique à squelette cyclohexane, de préférence le terpène d'orange, le terpène de citron ou le limonène.

4. Détergent selon l'une quelconque des revendications 1 à 3, **caractérisé en ce qu'**il présente 1,5 à 30 % en poids de la substance insoluble dans l'eau, dans lequel les pourcentages se rapportent au poids total du détergent.

5. Détergent selon l'une quelconque des revendications 1 à 4, **caractérisé en ce que** le composé d'acétoacétate est contenu à 2 à 25 % en poids dans le détergent, dans lequel les pourcentages se rapportent au poids total du détergent.

6. Détergent selon l'une quelconque des revendications 1 à 5, **caractérisé en ce que** le composé d'acétoacétate est l'éthylacétoacétate.

7. Détergent selon l'une quelconque des revendications 1 à 6, **caractérisé en ce que** l'autre substance amphiphile présente une solubilité dans l'eau de 2 à 128 g par litre et la substance est sélectionnée parmi le groupe comprenant des alcools en C₄-C₁₂, des cycloalcanols, des alcools phényliques, des acides gras à chaîne courte ou leur sels alcalins ou d'ammonium.

8. Détergent selon l'une quelconque des revendications 1 à 7, **caractérisé en ce que** l'autre substance amphiphile est contenue à 2 à 40 % en poids dans le détergent, dans lequel les pourcentages se rapportent au poids total du détergent.

9. Détergent selon l'une quelconque des revendications 1 à 8, **caractérisé en ce que** le tensioactif selon la revendication 1d) est contenu à 9 à 16 % en poids dans le détergent, dans lequel les pourcentages se rapportent au poids total du détergent.

10. Procédé de fabrication d'un détergent pour l'élimination de peintures et de laques indésirables de surfaces, **caractérisé en ce que** de l'eau ou un solvant à fonction hydroxy est présent(e) et un tensioactif anionique, cationique, amphotère et/ou non ionique y est dissous sous agitation à 10 à 90°C, une/des substance(s) insoluble(s) dans l'eau sont ajoutées en parallèle ou après l'addition de tensioactif et l'émulsion obtenue est ensuite transformée en une microémulsion optiquement transparente ou un système de nanophases par l'addition d'une autre substance amphiphile et d'un composé d'acétoacétate et des adjuvants sont éventuellement ajoutés à la fin du processus de mélange.

11. Procédé d'élimination de peintures et de laques indésirables de surfaces, **caractérisé en ce que** le détergent selon l'une quelconque des revendications 1 à 9 est appliqué sur la peinture indésirable ou la laque, agit, puis la peinture ou la laque est éliminée à l'eau, dans lequel la durée d'action pour les décapants pour graffitis est d'environ 10 secondes à environ 30 minutes, pour les décapants d'environ 20 minutes à environ 3 heures et pour les dissolvants d'environ 3 à environ 30 secondes.

12. Utilisation du détergent selon l'une quelconque des revendications 1 à 9 en tant que décapant pour graffitis, décapant ou dissolvant.
